# EUROPEAN PATENT APPLICATION

(11) **EP 2 444 007 A1**
(43) Date of publication of application: **25.04.2012**
(21) Application number: 10789028.7
(22) Date of filing: 27.05.2010
(51) Int. Cl.: A61B 17/04, A61B 17/3201

(54) **STITCH-REMOVAL PINCER**

(30) Priority: 15.06.2009 ES 200901415
(71) Applicant: Gutierrez Aramberri, Mikel Igor Carlos, 28033 Madrid (ES)
(72) Inventor: Gutierrez Aramberri, Mikel Igor Carlos, 28033 Madrid (ES)
(74) Representative: ABG Patentes, S.L.
(86) International application number: PCT/ES2010/000235
(87) International publication number: WO 2010/146195

(57) **Abstract**

The present invention relates to stitch-removal forceps used for removing suture stitches sewn with any type of non-absorbable suture. The stitch-removal forceps have a distal mechanism **characterized by** 3 elements: the wedge (2), an extension of the upper handle (1), the gripper (8), an extension of the upper portion (7) of the lower handle (3), and the cutter (4), an extension of the lower handle (3). These elements are operated using the handles (1) and (3) through only one pin (5), allowing the forceps to perform two independent functions.

Sutures are currently removed using two hands, one holding the scalpel blade and the other holding forceps pulling the suture thread for cutting and removing it.

The stitch-removal forceps allow performing this dual grasping/cutting function in a two-step action, removing the stitch.

## Description

### Field of the Art

The object of the patent is encompassed in the healthcare sector. Patients who have been surgically operated on or patients who have suffered from an incised wound are sutured with staples or with suture threads. The stitch-removal forceps are conceived to remove the suture threads from the skin with the movement of only one hand and leaving the other hand free.

### Prior State of the Art

Surgical approaches and incised wounds caused by traumas generally require a primary closure. This primary closure can be carried out with staples or suture threads, depending on several factors. One of the most important factors for determining with what material a wound must be closed is its location. Thus, surgical wounds or incisions located on the face, feet or hands are generally sewn with suture threads. Surgical approaches and incised wounds in children are also generally closed with suture threads instead of staples.

There are 2 types of suture threads, absorbable thread and non-absorbable thread. Since non-absorbable thread is made of a material which does not interact with the skin, it causes fewer skin reactions and is better tolerated, which is the main reason as to why it is the chosen method for closing wounds on the face, hands, feet and in children.

The object of the present invention is to remove suture threads sewn with any type of non-absorbable suture except staples.

In most hospitals today, this procedure for removing sutures from the skin is performed with forceps pulling the suture thread to enable, in turn, passing a scalpel blade between the skin and the thread, such that the scalpel blade faces the thread, cutting it. Thus, while the thread is cut with one hand, it is secured with the other and removed after cutting.

There are various surgical instrument elements intended for cutting or removing suture threads, although they do not have a dual function mechanism actuated with one hand such as the stitch-removal forceps. The dual function mechanism allows, in a first stroke of the forceps, securing the thread. At this time and before continuing with the bending of the fingers, the mechanism is provided with a change in intensity perceptible in the bending of the fingers, allowing the user to feel the start of the second function of the mechanism, the cut thus being made. Once the mechanism of the forceps has been completely actuated, the suture thread is firmly held by a portion of the mechanism and has been cut by the cutter (4), therefore removing the thread from the skin is possible, all with the same hand.

Another basic distinguishing feature with respect to the prior state of the art is the manner in which the mechanism of the forceps acts, assuring safety. Once the distal mechanism of the forceps is introduced between the skin and the suture thread, due to the design of the wedge (2), the distal portion of the upper handle (1), the thread itself tends to be lifted and separated from the skin. At this time the first function of the mechanism is performed, performing the first stroke of the handles (1) and (3) of the forceps until they (6) and (7) come into contact, and the thread is trapped. At this time, and before proceeding to the second function, a change in intensity in the bending of the fingers is perceptible, which allows repositioning, if desired, the distal mechanism if it is not holding the thread where the stitch is to be cut.

This first function of the forceps further allows, if necessary, trapping the thread and sliding the thread to the right or to the left of the wound so that the wedge (2) can be introduced to the right of the knot (operating standard to be taken into account which is detailed below).

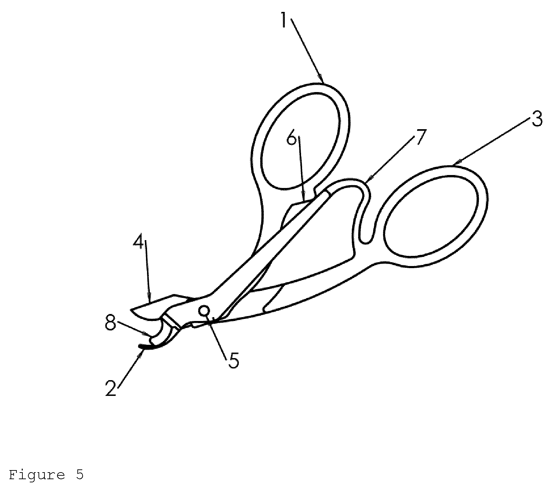

The other distinguishing feature is the cutting mechanism which is integrated in the distal mechanism of the forceps. It is a cut made with a cutter (4) in the vertical direction (not horizontal as in the patent from January 1983, of Lasner *et al,* patent number 4369787, which causes abrasions and lesions of the sutured wound) and on the previously secured and lifted thread. Furthermore, the stroke of the cutter (4) is less than the assembly of the mechanism (2) (8) which traps the thread, cutting the skin not being physically possible, since the cutter (4) does not protrude beyond the gripping mechanism (8), assuring safety (Fig. 5).

Several patents are found by performing a search in this field, of which the following standout:
1. US Patent 27 January 1981, Lasner, patent number 4246698.
2. US patent 9 July 1985, Lasner, patent number 4527331.
3. US patent 25 January 1983, Lasner *et al*, patent number 4369787.
4. US patent 13 September 1994, Suchart, patent number 5346500.

### Description of the Invention

### Technical Problem Raised

Two hands must be used to remove a suture thread from the skin. In one hand, generally the left, sterile forceps which allow lifting the thread are used, and a sterile scalpel blade is usually used (or sometimes scissors the edge of which has been molded to cut the threads) with the right hand. This current method can be especially dangerous, as in the case where suture threads in a small child must be removed, whose agitation, movement and ignorance with respect to the means can cause a new incised wound caused by the very sharp scalpel blade. Similarly, the healthcare personnel responsible for performing this task can also be injured (suffering an incised wound), and/or also be contaminated by the blood of the patient if he/she has an infection of any type or even be infected if the patient is a carrier of an infectious diseases such as hepatitis C or acquired immunodeficiency syndrome (AIDS), due to handling contaminated scalpel blades.

The stitch-removal forceps allow removing the suture threads with several advantages with respect to the instruments used today: only one hand is needed for its use due to its dual function mechanism. In addition, they improve the safety of the patient and of the healthcare personnel.

### Technical Advantages Provided by the Invention

### 1. Use with only one hand

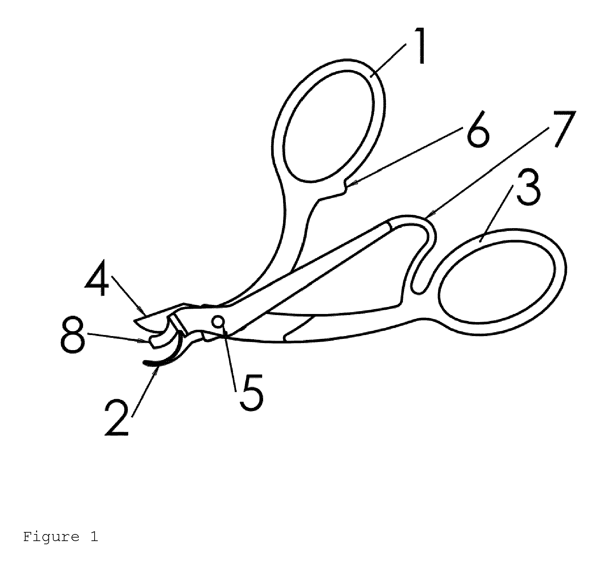

The stitch-removal forceps have been conceived for removing the suture non-absorbable threads. It has an acting mechanism which performs two functions triggered with only one hand. The forceps are actuated by means of the progressive bending of the fingers on the handles (1) and (3) (Figures 1, 2, 3 and 4).

### 2. Two linked but differentiated functions

Both functions are performed during the stroke of the forceps. The first of them consists of holding and securing the thread (Figures 5, 6 and 7), whereas the second function consists of cutting the thread, the first of the functions (Figures 8, 9 10 and 11 ) in turn being maintained. Once the entire stroke of the forceps is completed, the thread will be secured and cut, the thread is therefore removed from the skin. Despite being harmonically linked through the gesture of bending the fingers, both functions are separated by a change in intensity clearly perceptible in the bending force of the fingers.

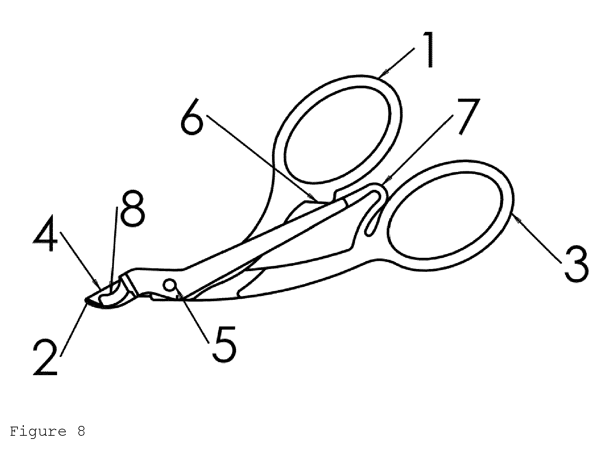

The following terms are used for subsequent explanation: the first portion of the stroke until the gripping effect is called stroke A. Stroke A is the distance separating the stop (6) of the upper handle (1) from the spring mechanism (7) integrated in the lower handle (3) (Figures 1, 8 and 9). Once both (6) and (7) are in contact the suture thread is secured between the wedge (2) and the gripper (8) (Figures 5, 6 and 7). The second portion of the stroke, in which the spring mechanism (7) and the lower handle (3) are in contact, is called stroke B (Figure 8). In this stroke the suture thread which is still secured by the wedge (2) and gripper (8) union is cut. The appearance of the forceps once strokes A and B are performed is seen in (Figures 8, 9 and 10).

Said change in intensity is caused by the spring effect (7) conceived within the design of the handles of the forceps (Figures 1, 8, 9 and 12) which will be explained in more detail below, increasing the pressure in the bending of the fingers being necessary so that the second function of the forceps (stroke B) starts.

This dual mechanism represents an essential difference with respect to any other tool described for the same function in the state of the art, since it allows cutting the thread wherever and whenever desired when it is actually secured.

Transferring said explanation to a practical example, if the child from whom the suture threads must be removed moves, the manner in which the thread is trapped can always be corrected before cutting it due to this change in intensity which allows a perceptible change between the function of securing and of cutting the thread.

### 3. Safety

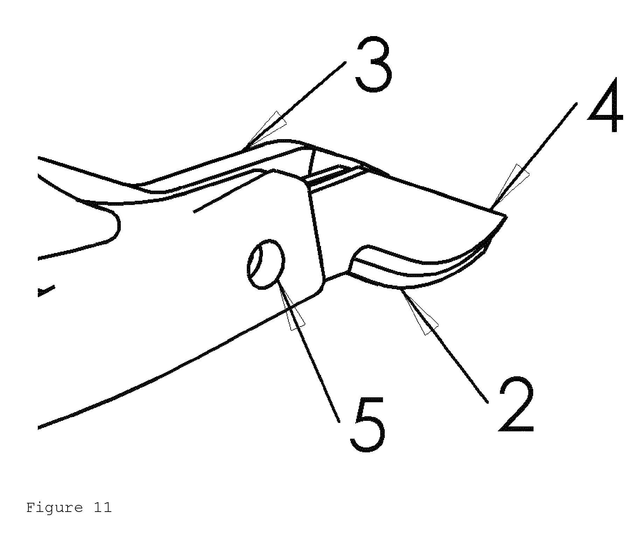

Another essential feature of the mechanism of the forceps for which they have been conceived is safety, both for the patient and for the healthcare personnel. In terms of the patient the cutter (4) integrated in the lower handle (3) has a stroke less than the gripper (8)and wedge (2) assembly of the forceps. Once the entire stroke thereof has been performed, the cutter (4) remains above the assembly which secures the thread, therefore cutting the patient is not possible (Figure 11). Similarly, the use thereof is simple and safe for the healthcare personnel.

### Brief Description of the Content of the Drawings

Figure 1 shows the complete profile of the forceps, wedge side, non-actuated mechanism.

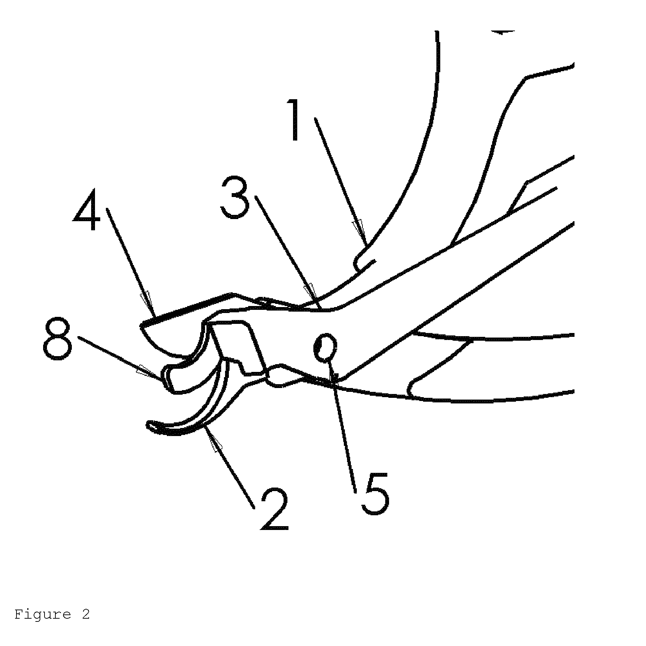

Figure 2 shows the detail of the distal non-actuated mechanism, wedge side.

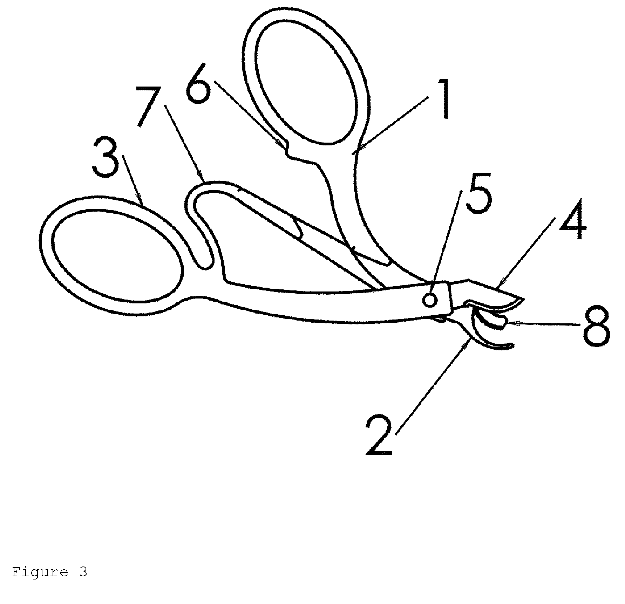

Figure 3 shows the complete profile of the forceps, cutter side.

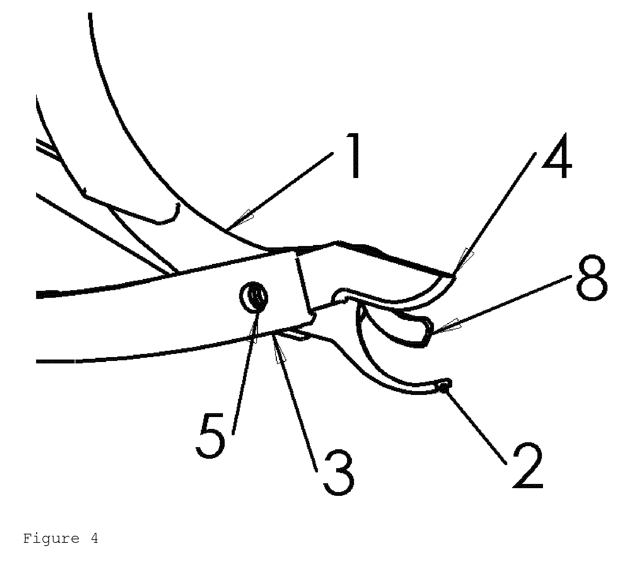

Figure 4 shows the detail of the distal non-actuated mechanism, cutter side.
Figure 5 shows the complete profile of the forceps, wedge side, holding the stitch.

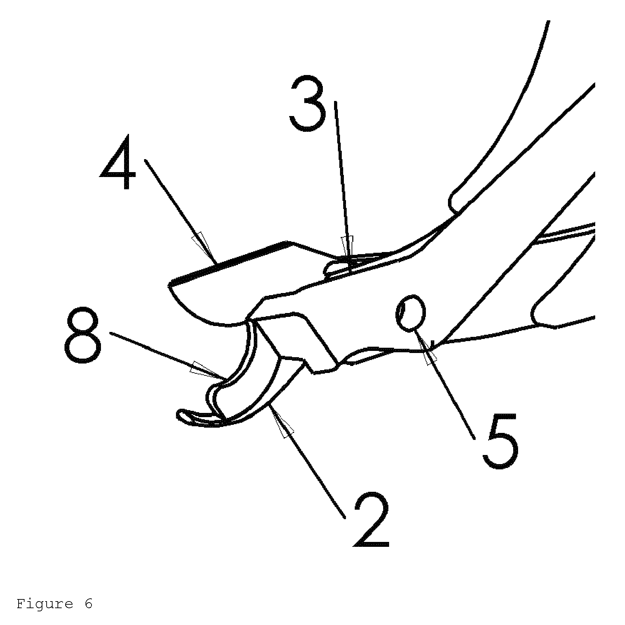

Figure 6 shows the detail of the distal mechanism holding the stitch, wedge side.

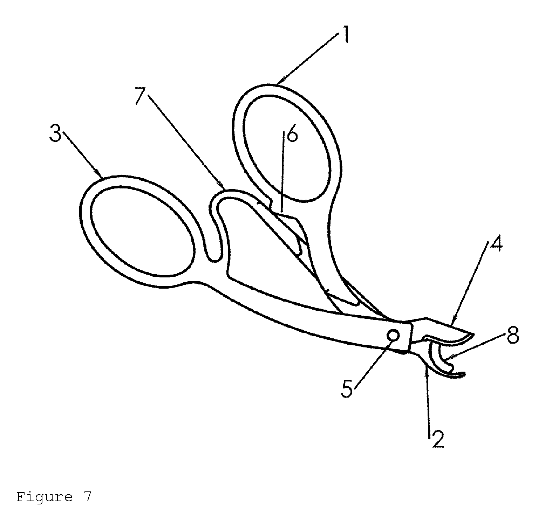

Figure 7 shows the complete profile of the forceps, cutter side, holding the stitch.
Figure 8 shows the complete profile of the forceps with the completed actuated mechanism, wedge side.

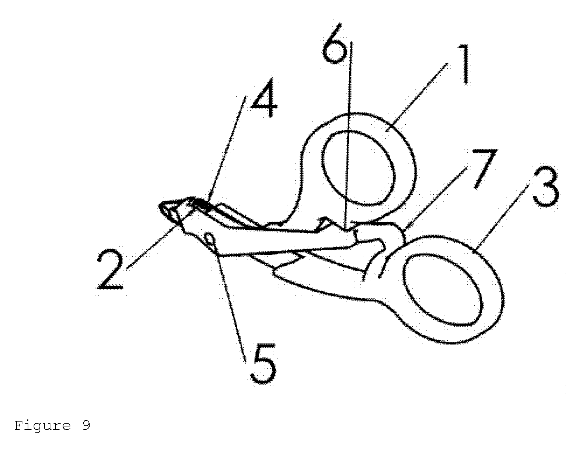

Figure 9 shows the 3D reconstruction of the forceps with the completely actuated mechanism, wedge side.

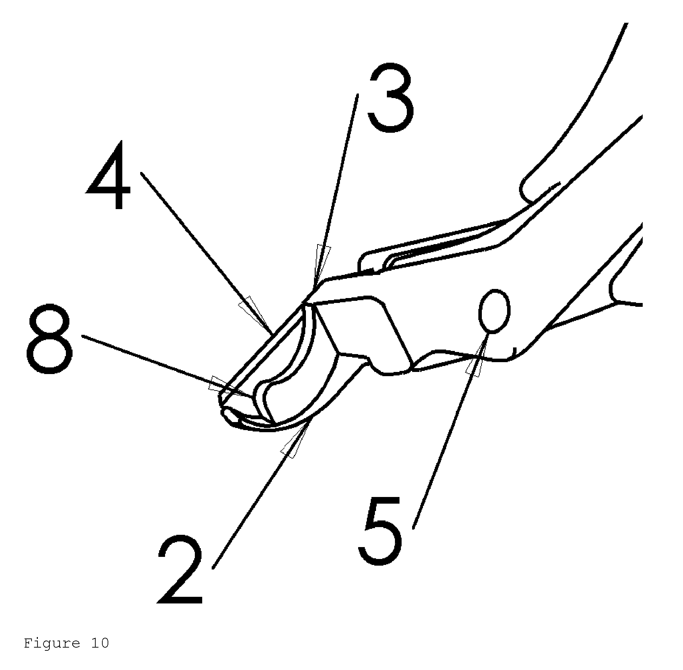

Figure 10 shows the detail of the completely actuated distal mechanism, wedge side.
Figure 11 shows the detail of the completely actuated distal mechanism, cutter side.

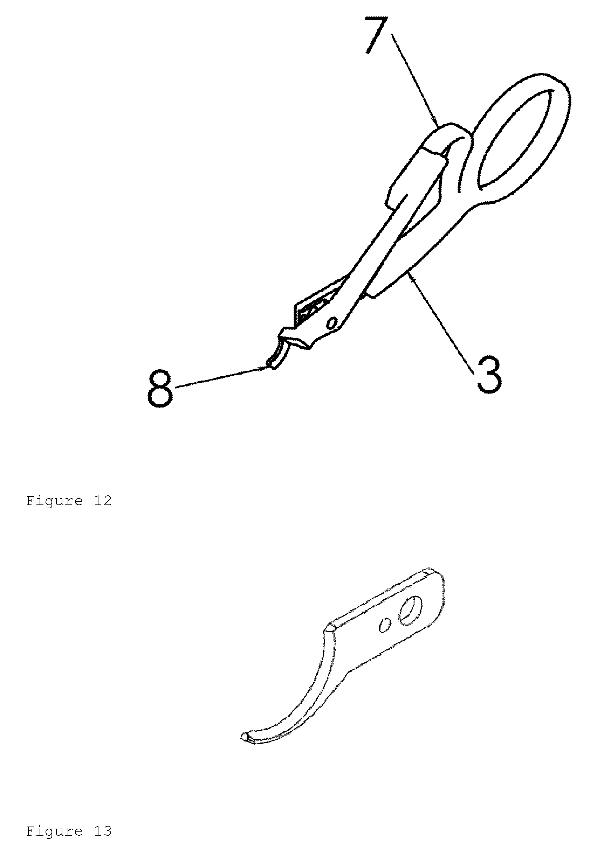

Figure 12 shows the 3D reconstruction of the lower handle (3) with the gripper (8) and the integrated spring mechanism (7).
Figure 13 shows the 3D reconstruction of the wedge.

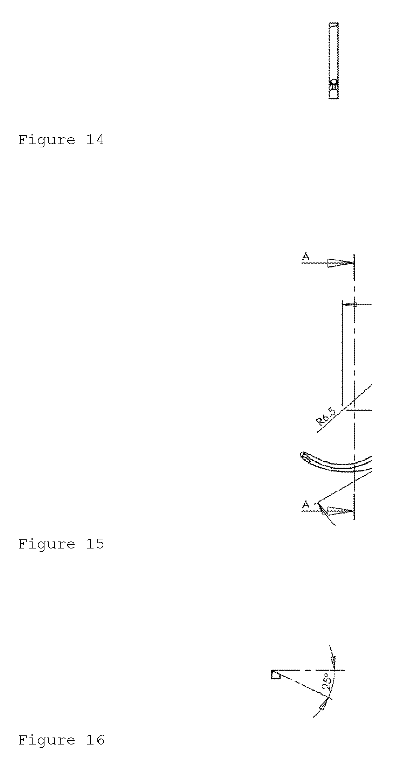

Figure 14 shows the front of the wedge where a blunt triangular tip which facilitates the passage between the skin and the thread can be seen.
Figure 15 shows the distal portion of the wedge in profile, through where section AA which is seen in Figure 16 is made.
Figure 16 shows section AA of the wedge where the 25° angle which will allow lifting the stitch after pressing the gripper to complementary 65° is seen, thus facilitating the lifting of the stitch and the cutting by the cutter (4).

### Detailed Description of the Invention

The stitch-removal forceps have at least two scissor-type actuating handles, an upper handle (1) and a lower handle (3), which are articulated and pivot about a common pin (5) and which are actuated with the fingers of only one hand actuating a dual function distal mechanism. These elements are described below:

### 1. Upper handle (1) and its distal mechanism: the wedge (2) (Figures 1 and 2)

The upper handle (1) incorporates the structure that wedges and lifts the thread in the distal mechanism, the wedge (2). This structure is in the form of an assembly similar to a jai alai glove. Starting from the overall and profile view, the structure has been distally angled 30 degrees downwards, which allows a better leading position of the wrist of the healthcare personnel using the forceps and a more comfortable and efficient functionality. Specifically, it can be observed that the tip of the wedge (2) ends in a pointed shape but is completely blunt so as to not prick or harm the skin, which facilitates the entrance of the forceps. The securing mechanism for securing the wedge (2) to the upper handle (1) consists of the fitting thereof in 2 openings, one which further acts as a movement pin, and the other which is introduced in the complementary cylinder of the upper handle (1) and confers additional stability to said functional unit for the mechanics of the forceps. Another specific feature of the wedge (2) is its width of preferably 1 mm but is not limited to this value, and its tapering which allows a more efficient initial sliding between the skin and the thread and subsequently a better lifting of the thread, since on many occasions the thread is buried rather deep in the skin. The wedge has a 25° angle when viewed from the front (Figures 13, 14, 15 and 16). This facilitates that, once the thread is secured with the gripper (8), the angle of which is complementary, the thread is oriented upwards, making the cutting easier.

### 2. Lower handle and its distal mechanism: the gripper (8) and the cutter (4) (Figures 1, 2, 3 and 4)

The lower handle is formed by an upper extension (7) conferring the property of a spring to the forceps. This upper extension incorporates the gripper (8) in its distal portion, which allows, once the stop (6) of the upper handle (1) and the spring (7) come into contact after having performed the first stroke A of the forceps, the suture thread to be trapped within the securing area of the thread between (2) and (8), and to be prepared to be cut.

This design has thus been conceived by taking into account the manufacturing process, given that a single mold is thus necessary to obtain the spring effect integrated within the lower handle. However the essential mechanism object of the patent is the dual function of the distal mechanism, and the effect of the change in intensity between stroke A and B can also be obtained through a spring integrated between the handles of the forceps close to the pin.

The gripper (8) has particular features which must be highlighted: it has a curvature which is perfectly adapted to the wedge and the width is in turn preferably 1 mm, although it is not limited to this value, such that the fitting between both mechanisms is maximum and assures that the suture thread is trapped between the wedge (2) and the gripper (8). Thus, once the first part of the mechanism (stroke A) is actuated, the suture thread is well secured. Another essential feature of the gripper (8) is that its length with respect to the wedge (2) is less, which allows the user to have visual control at all times, thus being able to position the knot of the suture thread in the correct area, which is to the left of the area of the gripper between (2) and (8), for the proper actuation of the forceps. The gripper in turn has a 65° angle in the lower securing area contacting the wedge (Figures 2 and 6) complementary to the 25° of the wedge (Figures 13, 14, 15 and 16) to better secure the thread and direct the thread upwards, which will facilitate cutting once secured. In a particular embodiment, the gripper (8) and the wedge (2) working together to secure the thread have a toothed profile to facilitate a better grip.

The lower handle (3) of the forceps incorporates the cutter (4), which allows cutting the suture thread during part B of the stroke of the bending of the fingers. As discussed above, this handle is attached to the upper portion (7) to confer a change in intensity perceptible in the hand upon changing between stroke A and stroke B which allows knowing when the cutting function starts and allows it to be when and where the user so desires. Similarly, it confers it with the elasticity necessary so that once the entire stroke is performed, the forceps return to their base position to be able to be used for taking out the next suture thread. The materials will be detailed more in depth in the embodiment section.

The cutter (4) has been angled 30 degrees towards the leading direction of the forceps (patient's skin) in the intermediate portion thereof such that the blade fits with the securing mechanism for securing the thread (2) and (8) and allows cutting the thread when part B of the stroke is performed. To secure the cutter (4) to the lower handle (3), the proximal portion has a primary structural slot and at least two holes, the movement pin acting on one, and the other being introduced in the complementary cylinder of the lower handle (3) and conferring said functional unit an even better stability for the mechanics of the forceps, as occurs between the upper handle (1) and the wedge (2). A possible subsequent modification of the cutter (4) can be to reduce the length of the blade, making it the same size as the gripper, since the portion of the thread secured by the gripper (8) is the portion to be cut. The end of the distal tip of the cutter (4) can be blunt like the shape of the gripper (8). The safety of the forceps is thus further enhanced, i.e., it only cuts on the lower face thereof which is where a cut is really needed.

The mechanism described can be adapted to forceps with eyes allowing the handling thereof from outside a joint, the abdominal cavity or any part of the human body, and through an elongated handle transmitting the functionality to the distal mechanism through the skin, muscles, joint capsule or any other anatomical structure; it can be used in handling suture threads within the human body as the result of viewing with arthroscopic and/or endoscopic techniques.

### Materials:

The body will be made of polymer and the injection process of plastic. The most common plastic which could be used in these cases is polypropylene, but POM (polyoxymethylene) or ABS (acrylonitrile butadiene styrene) or in general, any other material susceptible to being used in an injection molding process could be chosen. The cutter (4) and the distal mechanism can be made of mild carbon steel, the most common being F-500 series steel, such as F-515 steel.

Another embodiment possibility is to make the distal mechanism with the materials described above, i.e., from F-500 series steel, the body is made of an injected plastic and for the change in intensity effect perceptible in the bending of the fingers a spring causing it can be included, or a physical stop of a part can be included, which can in turn be released with another finger and thus the dual function can be included.

As long as the purpose of the forceps is the use thereof in a medical environment, choosing the materials will need to be based on the regulations in force.

The stitch-removal forceps are conceived for a disposable use, i.e., one use for each patient, maximally assuring the sterility of each procedure. However and depending on the materials from which it is made, the forceps can be resterilized after each use.

### Operation:

1. Basic operation standard: For the proper use of the forceps, they must simply be inserted between the skin and the thread, and to the right of the knots of the thread, such that once both functions of the forceps are carried out, the thread can be removed without the knots catching on the skin when pulling the thread.
2. The forceps are secured with only one hand, stroke A of the forceps is performed once the thread has been wedged between (2) and (8), until the stop between (6) and (7), which allows securing it, (Figures 1 and 5 )
3. Stroke B of the forceps continues until (7) and (3) come into contact, cutting the suture thread. (Figure 8)
4. Maintaining strokes A and B, i.e., with the thread secured and cut, the suture thread is removed from the skin. (Figures 9 and 10)
5. The bending of the fingers is released, the removed thread is left in a gauze and the process can be restarted with the next thread by returning the forceps to their initial position as a result of the spring effect caused by the elasticity of (7).

### Industrial application:

The forceps are conceived for a single use due to their sterile nature.

They can contribute to the removal of sutures from patients in many hospitals, therefore the mass production thereof is possible.

## Claims

1. Stitch-removal forceps for removing sutures sewn with any non-absorbable suture of the type comprising:
• two scissor-type actuating handles (1) (3) pivoting about a common pin (5) and actuated with the fingers of only one hand, and acting on a dual function distal mechanism, **characterized in that**:
• the lower handle (3) in the normal actuation position of the stitch-removal forceps is attached at its middle and upper portion to a spring mechanism (7) providing a spring effect, which produces a change in intensity in the closing movement when a stop (6) incorporated in the upper handle (1) contacts the spring (7) in the actuation of the handles, such that an increase in the resistance to the closing movement which can be overcome is produced from that point and also upon releasing the handles, they return to their initial open position, the spring (7) consisting of a third handle which also pivots about the pin (5) and which is attached at the middle area to the lower handle (3) by means of a flexible area,
• the upper handle (1) incorporates in its distal portion a wedge (2) with a rounded-shape in its area of contact with the skin during use and a tip which is in turn triangular and blunt to facilitate the passage between the skin and the thread and having a tapering from the tip towards the rear area thereof and a 25° cross-sectional angling on the longitudinal axis thereof which, together with the radius of curvature, allows and facilitates lifting the thread,
• the spring mechanism (7) incorporates in its distal portion a gripper (8) with a radius of curvature which is perfectly adapted to the radius of curvature of the wedge (2) and with a cross-sectional angling on its longitudinal axis complementary to that of the wedge (2) to grip the thread between both parts when the stop (6) and the spring (7) come into contact in the closing movement of the handles (1) (3), the length of the gripper (8) being less than the length of the wedge (2) to facilitate viewing the entrance of the thread into the securing area thereof,
• the lower handle (3) incorporates in its distal portion a cutter (4) for cutting the thread which is secured between the wedge (2) and the gripper (8) when the closing movement of the handles (1) (3) continues, overcoming the resistance provided by the spring mechanism (7), the cutting blade of the cutter (4) being located higher than the lower portion of the wedge (2) when the cut is finished to prevent cutting the skin.

2. The stitch-removal forceps according to claim 1, wherein the upper handle (1) and lower handle (3) are attached to the distal mechanism through the means having a greater length sufficient for allowing the use thereof in an arthroscopic and/or endoscopic surgery.

3. The stitch-removal forceps according to claim 1 or 2, wherein the gripper (8) and the wedge (2) working together to secure the thread have a complementary toothed profile to provide a better grip.

4. The stitch-removal forceps according to claim 1 or 2, wherein the gripper (8) and the wedge (2) working together to secure the thread have a profile with a smaller radius of curvature or even a lance-like straight profile to facilitate entering between the skin and the suture thread if they are buried.

5. The stitch-removal forceps according to claim 1 or 2, wherein the cutter (4) has a length equal to or less than the length of the wedge (2) for cutting the suture thread and it further has only one cutting blade on the lower surface, and the end of the distal tip of the cutter (4) is blunt.

6. The stitch-removal forceps according to claim 1 or 2, wherein the spring effect is produced by incorporating a spring close to the pivoting pin (5) acting between the handles (1) and (3) instead of the spring (7).
